# EUROPEAN PATENT APPLICATION

(11) **EP 4 279 066 A1**
(43) Date of publication of application: **22.11.2023**
(21) Application number: 22739722.1
(22) Date of filing: 13.01.2022
(51) Int. Cl.: A61K 9/08, A61K 47/12, A61K 47/02, A61K 9/00, A61K 38/16, A61P 27/02

(54) **OPHTHALMIC COMPOSITION INCLUDING TANFANERCEPT AND EXHIBITING STABILITY WITHOUT USE OF STABILIZER**

(30) Priority: 14.01.2021 KR 20210005584
(71) Applicant: Hanall Biopharma Co., Ltd., Daejeon 34344 (KR)
(72) Inventor: PARK, Seung Kook, Seoul 06374 (KR); AHN, Hyea Kyung, Yongin-si Gyeonggi-do 16938 (KR); JUNG, Mi Jin, Suwon-si Gyeonggi-do 16493 (KR); SHIM, Hye Eun, Seoul 08774 (KR)
(74) Representative: Plasseraud IP
(86) International application number: PCT/KR2022/000661
(87) International publication number: WO 2022/154529

(57) **Abstract**

The present invention relates to a stable ophthalmic composition comprising tanfanercept, which does not use a stabilizer, and methods of preparing and using the composition. According to the present invention, it was found that the use of a stabilizer such as histidine or sucrose causes the generation of impurities such as tanfanercept-derived acidic/basic variants, and affects biological activity. The ophthalmic pharmaceutical composition according to the present invention may control pH rather than excluding the use of such a stabilizer to significantly reduce the generation of impurities under not only a refrigerated storage condition but also accelerated conditions and a stress condition, thereby preparing a stable tanfanercept ophthalmic composition.

## Description

### [Field of Invention]

The present invention relates to a stable ophthalmic composition comprising tanfanercept without using a stabilizer, and methods of preparing and using the composition.

### [Background of Invention]

In general, therapeutic agents for topical administration to eyes are formulated in the form of either a liquid or a gel, and stably maintained in an aseptic state until administration. These ophthalmic solutions contain buffers, various surfactants, stabilizers and isotonic agents, and help to make an ophthalmic composition more comfortable for users. The stability of the solution is particularly important in the efficiency and commercialization of the solution. Solution stability may vary according to not only the interaction between all compounds present in a formulation, but also a temperature and pH.

A protein-containing pharmaceutical composition is physicochemically denatured under conditions that are not optimal. Particularly, factors such as the concentration of protein, the type of buffer, the type and concentration of stabilizer, the type and concentration of organic cosolvent, the concentration of salt, pH, temperature, and contact with the air significantly influence the oxidation, deamidation, isomerization and polymerization of a protein. Such denaturation may reduce physiological activity by generating an aggregate, fragment and isomer of a protein.

Meanwhile, a tumor necrosis factor (TNF-α) is a cytokine produced by many cell types, including monocytes and macrophages. TNF-α is associated with a variety of other human immune diseases, including infections, autoimmune diseases, sepsis and transplant rejection. Since the overexpression of TNF-α leads to negative consequences in human diseases, a therapeutic agent has been designed to control or attenuate the activity of TNF-α. Accordingly, antibodies binding to TNF-α to neutralize have been developed and sold as various protein preparations. However, due to a large molecular weight, such an anti-TNF-α antibody preparation has a limitation in that the preparation cannot efficiently reach an inflammatory disease caused in a local area. Therefore, the present applicants have developed a polypeptide molecule suitable for the treatment of a local inflammatory disease due to a small size and high activity. The TNF-α inhibitor of the present invention, which is a modified human TNF receptor-1 polypeptide, tanfanercept, is disclosed in Korean Patent Laid-Open Publication No. 2012-0072323, filed by the present applicant, and its use for treating xerophthalmia is disclosed in Korean Patent Laid-Open Publication No. 2013-0143484.

### [Related Art Documents]

### [Patent Documents]

Korean Patent Laid-Open Publication No.2012-0072323
Korean Patent Laid-Open Publication No.2013-0143484

### [Summary of Invention]

### [Technical Problem to be Solved]

A protein composition may have a shorter shelf life compared to chemically synthesized drugs, and physicochemical impurities such as charge variants or aggregates caused during the storage period may be generated, thereby degrading biological activity.

The present invention is directed to providing a stable ophthalmic composition, which includes tanfanercept, without using a stabilizer.

### [Technical Solution]

The present inventors conducted various studies on the stability of tanfanercept to develop an ophthalmic composition capable of minimizing the generation of tanfanercept-derived impurities (acidic/basic variants) when stored not only under a refrigerated storage condition but also under accelerated and stress conditions. Particularly, the present inventors conducted various formulation studies, including a buffer, an isotonic agent, a pH range and a functional excipient. As a result, the present inventors have found that the use of stabilizers such as histidine and sucrose allows acidic/basic variants to be generated even at a specific pH such that the stability of the tanfanercept composition is affected. Therefore, the present invention is to provide a stable tanfanercept ophthalmic composition at pH 5.0 to pH 6.5, which does not substantially comprise a stabilizer.

Specifically, the present invention provides a stable tanfanercept-containing ophthalmic composition, which comprises tanfanercept and a buffer system of pH 5.0 to pH 6.5, and does not substantially comprise a stabilizer.

Tanfanercept is a TNFRI variant disclosed in Korean Laid-Open Publication No. 2013-0143484, and is represented by an amino acid sequence including the amino acid modification of L68V/S92M/H95F/R97P/H98G/K161N in the amino acid sequence (TNFRI171) consisting of amino acids 41 to 211 of the amino acid sequence of wild-type TNFRI.

Since tanfanercept is a polypeptide consisting of a total of 171 amino acids, like general protein-containing pharmaceutical compositions, one of the most important issues in drug development to ensure stability until the composition is administered to a patient and exhibits the best drug effect.

To develop a formulation ensuring the stability of tanfanercept, the present inventors first examined the storage stability of tanfanercept, and as a result, it was observed that tanfanercept forms a charge variant during storage (Experimental Example 1). The term "charge variant" used herein means that the charge of a protein or polypeptide is changed by modification from its natural state. In some examples, charge variants are more acidic than the original protein or polypeptide; that is, have a lower pI value than the original protein or polypeptide. In another example, charge variants are more basic than the original protein or polypeptide, that is, have a higher pI value than the original polypeptide. Such modifications may be caused by the result of engineering or natural processes, for example, oxidation, deamination, C-terminus processing of a lysine residue, the formation of N-terminus pyroglutamate, and non-enzymatic glycosylation. In some examples, protein or polypeptide charge variants are glycoproteins having charges changed by the addition of a glycan attached to the protein, for example, sialic acid or a derivative thereof, compared to a parent glycoprotein. The "tanfanercept charge variant" used herein is a material in which the charge of tanfanercept is changed by tanfanercept being modified from its natural state.

Generally, since it is well known that charge variants generally cause degraded drug activity, it is necessary to manage the production amount of a charge variant to a certain level or less. Therefore, the present inventors confirmed that the generation of impurities such as charge variants may be minimized using components that can be used as ophthalmic stabilizers, and sucrose and histidine may be primarily included in the composition (Experimental Example 2). However, in additional research to determine an appropriate pH of the tanfanercept-containing composition, it was confirmed that the production rate of the charge variant is high even when using stabilizers at a specific pH, and a method of reducing the production rate of the charge variant to the lowest level includes increasing the production rate of the charge variant, and adjusting pH to 5.0 to 6.5 without using a stabilizer (Experimental Example 3).

Therefore, the present invention provides a tanfanercept-containing ophthalmic composition, which comprises tanfanercept and a buffer system of pH 5.0 to pH 6.5, and does not substantially comprise a stabilizer.

Tanfanercept is preferably comprised in the composition at an appropriate content, because the higher the content, the higher the content of impurities such as an aggregate. In the tanfanercept-containing ophthalmic composition of the present invention, tanfanercept may be comprised at a content of 0.01 to 10 %(w/v), for example, 0.01 to 8 %(w/v), 0.01 to 6 %(w/v), 0.01 to 4 %(w/v), 0.01 to 2 %(w/v), 0.01 to 1 %(w/v), 0.02 to 1 %(w/v), 0.05 to 0.8 %(w/v), 0.1 to 0.7 %(w/v), or 0.2 to 0.6 %(w/v). In consideration of commercial purposes, tanfanercept may be included in the composition at a content of 0.25 %(w/v), 0.5 %(w/v), 1 %(w/v), 2 %(w/v), 3 %(w/v), 4 %(w/v), 5 %(w/v), 6 %(w/v), 7 %(w/v), 8 %(w/v), 9 %(w/v), or 10 %(w/v). The content of tanfanercept may vary according to the type and severity of a disease of a patient to be administered.

The tanfanercept-containing ophthalmic composition according to the present invention comprises a buffer system of pH 5.0 to pH 6.5. It is sufficient for the buffer system to have a pH of pH 5.0 to pH 6.5, and all numerical ranges included in the range from pH 5.0 to pH 6.5, for example, a buffer system of pH 5.0 to pH 6.0, a buffer system of pH 5.5 to pH 6.5, a buffer system of pH 5.5 to pH 6.0, and a buffer system of pH 5.8 to pH 6.3 are included in the scope of the present invention. In one embodiment of the present invention, the tanfanercept-containing ophthalmic composition according to the present invention comprises a buffer system of pH 5.0 to pH 6.0. In another embodiment of the present invention, the tanfanercept-containing ophthalmic composition according to the present invention comprises a buffer system of pH 5.5 to pH 6.0.

In the tanfanercept-containing ophthalmic composition according to the present invention, a method of implementing a buffer system is well known to those of ordinary skill in the art. The buffer system of pH 5.0 to pH 6.5 may comprise one or two or more buffers selected from the group consisting of a phosphate buffer, a histidine buffer, an acetate buffer, a succinate buffer, a citrate buffer, a glutamate buffer and a lactate buffer. Regardless using any buffer system, if the buffer system satisfies the condition of pH 5.0 to pH 6.5, it is confirmed that the stability of tanfanercept can be ensured. However, the use of a specific buffer system may be relatively desirable. According to the following examples, compared to an acetate buffer, it was confirmed that a citrate buffer is relatively advantageous in terms of controlling the generation of aggregates or charge variants (Experimental Example 4). Accordingly, in one embodiment of the present invention, the buffer system may be a citrate buffer-containing buffer system, for example, a citrate buffer system or a citrate-phosphate buffer system, but the present invention is not limited thereto. The buffer included in the buffer system may consist of a combination of conjugate acid-conjugate base to enhance a buffering effect. For example, in one embodiment of the present invention, the buffer system includes a citrate buffer, and the citrate buffer includes trisodium citrate (conjugate base) and citric acid (conjugate acid).

The buffer system of the present invention comprises a 5mM to 50 mM buffer, for example, a 10 mM to 30 mM buffer.

The ophthalmic composition of the present invention does not substantially comprise a stabilizer. Here, the stabilizer means an additional component comprised in a formulation to prevent a decrease in chemical and physical stability or biological activity of tanfanercept used as an active ingredient. For example, to inhibit the aggregation of a protein in the ophthalmic composition, it is well known that the stabilization of a protein may be ensured using a saccharide such as sucrose or mannitol, or an amino acid-based stabilizer such as proline, arginine, glycine, lysine or methionine. The present invention does not substantially comprise a stabilizer since it had been found from the following embodiment that, unlike the usual cases, such a stabilizer has an adverse effect on the stability of tanfanercept. The buffer or isotonic agent described in the present invention is not included in the stabilizer.

The stabilizer is "not substantially comprised" or "substantially free" means that it is comprised at less than 0.1 %(w/v), 0.05 %(w/v), 0.03 %(w/v), 0.02 %(w/v), 0.01 %(w/v), 0.005 %(w/v) or 0.001 %(w/v), and most preferably, not comprised at all.

The osmolality of the ophthalmic composition may be 260 to 320 mOsm/kg.

The ophthalmic composition according to the present invention may further comprise an isotonic agent in addition to tanfanercept as an active ingredient and a buffer system. The isotonic agent is used to adjust the osmotic pressure of the ophthalmic composition according to the present invention. In the present invention, the isotonic agent is comprised such that the ophthalmic composition according to the present invention has an osmolality of 260 to 320 mOsm/kg. The osmotic pressure is determined by measuring the number of particles dissolved per unit of water. In solutions, as the number of solute particles is lowered in proportional to the unit number of water (solvent), a low-osmotic pressure solution is less concentrated. When solutions with different solute concentrations are separated using a semi-permeable membrane (membrane permeable to only solvent molecules), osmosis in which solvent molecules cross the membrane from a low concentration to a high concentration to form a concentration equilibrium occurs. A pressure driving this movement is called osmotic pressure, and controlled by the number of "particles" of a solute in a solution. A solution containing the same concentration of particles and applying the same osmotic pressure is called isosmotic. If a hypotonic or hypertonic solution is placed in an eye, it may damage the eye, and thus an isotonic solution for a drug used on eyes is required. As an isotonic agent, in the present invention uses sodium chloride. In the ophthalmic composition according to the present invention, the content of the isotonic agent may be 0.5 to 1 %(w/v). In the ophthalmic composition according to the present invention, the concentration of the isotonic agent may be 100 to 150 mM.

In one embodiment of the present invention, the ophthalmic composition according to the present invention consists of tanfanercept, a buffer system of pH 5.5 to pH 6.0, an isotonic agent and water. In one embodiment, the ophthalmic composition according to the present invention consists of tanfanercept, a buffer system of pH 5.5 to pH 6.0 containing a citrate buffer, sodium chloride and water.

The tanfanercept-containing ophthalmic composition according to the present invention is very stable even under accelerated or stress conditions.

The tanfanercept-containing ophthalmic composition according to the present invention may have charge variants in an amount of 20% or less after storage for 6 months under accelerated conditions.

The tanfanercept-containing ophthalmic composition according to the present invention may have basic variants in an amount of 10% or less after storage for 6 months under accelerated conditions.

The tanfanercept-containing ophthalmic composition according to the present invention may have acidic variant in an amount of 10% or less after storage for 6 months under accelerated conditions.

The tanfanercept-containing ophthalmic composition according to the present invention may have tanfanercept charge variants in an amount of 20% or less after storage for 36 months under a long-term storage condition.

The tanfanercept-containing ophthalmic composition according to the present invention may have tanfanercept basic variants in an amount of 10% or less after storage for 36 months under a long-term storage condition.

The tanfanercept-containing ophthalmic composition according to the present invention may have tanfanercept acidic variants in an amount of 10% or less after storage for 36 months under a long-term storage condition.

As the physicochemcal and biological stability of tanfanercept is improved, the ophthalmic, pharmaceutical composition according to the present invention can be administered to a patient suffering from an ophthalmic disease mediated by TNF such as xerophthalmia according to a conventional method such as instillation.

### [Effect of Invention]

According to the present invention, it was found that the use of a stabilizer such as histidine or sucrose causes the generation of impurities such as tanfanercept-derived acidic/basic variants, and affects biological activity. The ophthalmic pharmaceutical composition according to the present invention may control pH rather than excluding the use of such a stabilizer to significantly reduce the generation of impurities under not only a refrigerated storage condition but also accelerated conditions and a stress condition, thereby preparing a stable tanfanercept ophthalmic composition.

### [Brief Description of Drawings]

FIG. 1 shows the result of isoelectric focusing (IEF) of tanfanercept after storage at 37 °C for 0 to 4 weeks;
FIG. 2 shows the result of IEX-HPLC analysis of tanfanercept after storage at 37 °C for 0 to 4 weeks;
FIG. 3 shows the result of IEF analysis for charge variants;
FIG. 4 shows the result of IEX-HPLC analysis for charge variants;
FIG. 5 shows the changes of main peak in IEX-HPLC in a control group not comprising a stabilizer, and stabilizer screening solution groups respectively comprising methionine, glycine, histidine hydrochloride and sucrose as a stabilizer;
FIG. 6 shows the changes of acidic variants in IEX-HPLC in a control group not comprising a stabilizer, and stabilizer screening solution groups respectively comprising methionine, glycine, histidine hydrochloride and sucrose as a stabilizer;
FIG. 7 shows the changes of basic variants in IEX-HPLC in a control group not comprising a stabilizer, and stabilizer screening solution groups respectively comprising methionine, glycine, histidine hydrochloride and sucrose as a stabilizer;
FIG. 8 shows the result of analyzing the stability of tanfanercept ophthalmic compositions prepared under conditions of various pHs and stabilizers after storage at 40 °C for 4 weeks by variants in RP-HPLC;
FIG. 9 shows the stability of tanfanercept ophthalmic compositions prepared under conditions of various pHs and stabilizers after storage at 40 °C for 4 weeks, represented by the changes of main peak in IEX-HPLC;
FIG. 10 shows the stability of tanfanercept ophthalmic compositions prepared under conditions of various pHs and stabilizers after storage at 40 °C for 4 weeks, represented by the changes of acidic variants in HEX-HPLC;
FIG. 11 shows the stability of tanfanercept ophthalmic compositions prepared under conditions of various pHs and stabilizers after storage at 40 °C for 4 weeks, represented by the changes of basic variants in HEX-HPLC;

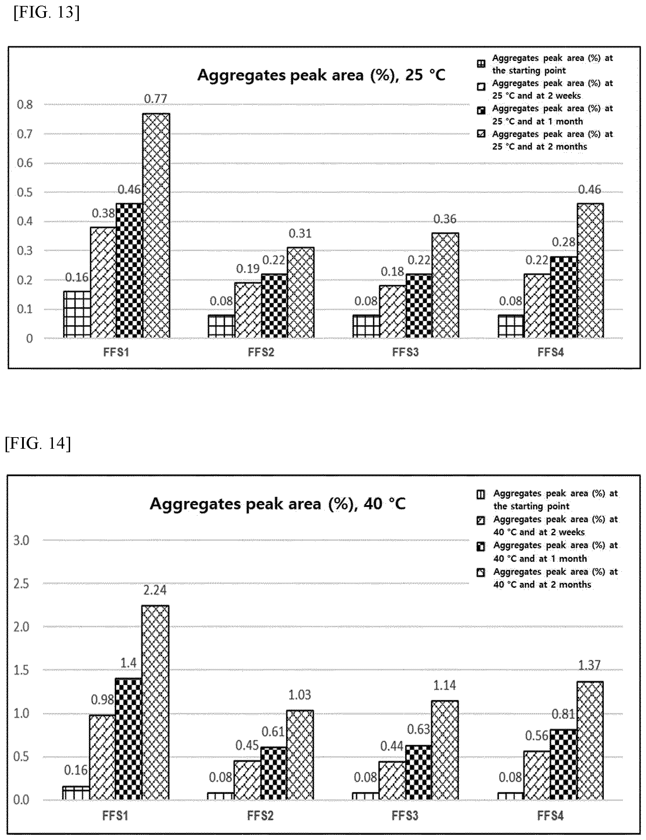

FIGS. 12 to 14 show the results of SEC-HPLC analysis performed while four types of tanfanercept ophthalmic compositions are stored at 4 °C, 25 °C and 40 °C, respectively;
FIGS. 15 to 17 show the result of analyzing acidic variants using IEX-HPLC performed while four types of tanfanercept ophthalmic compositions are stored at 4 °C, 25 °C and 40 °C, respectively;
FIGS.18 to 20 show the result of analyzing basic variants using IEX-HPLC performed while four types of tanfanercept ophthalmic compositions are stored at 4 °C, 25 °C and 40 °C, respectively;
FIG. 21 shows the stability of tanfanercept ophthalmic compositions by the result of analyzing aggregates using SEC-HPLC; and
FIGS. 22 and 23 show the stability of tanfanercept ophthalmic compositions by the result of analyzing charge variants using IEX-HPLC.

### [Detailed Description of Preferred Embodiments of Invention]

Advantages and features of the present invention and methods for accomplishing the same will be clarified with reference to the following preparative examples, illustrative examples and experimental examples. However, these examples are provided only to facilitate understanding of the present invention and are not to be construed as limiting the invention.

### Experimental Example 1: Assay of tanfanercept charge variant

Generation of charge variants may influence drug activity, stability and safety and, therefore, the present inventors first analyzed the charge variants of tanfanercept. More particularly, after storing tanfanercept at 37 °C for 4 weeks, isoelectric focusing (IEF) and IEX-HPLC analysis were implemented.

### Isoelectric focusing method

10 µg per well was loaded in a gel at pH 3.0 to pH 7.0, followed by conducting electrophoresis at 100V for 1 hour, 200V for 1 hour and then 500V for 30 minutes. The product was fixed with 12% trichloroacetic acid for 30 minutes and then stained with Coomassie blue.

### IEX-HPLC (ion exchange-high performance liquid chromatography)

Ion exchange-high performance liquid chromatography (IEX-HPLC) is used to separate protein using an ion exchanger on the basis of affinity to a fixed phase in a column, which is relevant to net charge of the protein. The present experimental method was conducted using a cation exchange column, a temperature controller (set to 25 °C), an automatic sampler (set to 4 °C), a UV detector driven at 280 nm and a high performance liquid chromatography (HPLC) system that may maintain a flow rate of 0.7 mL/min.

### Separation and purification of charge variants

In order to separate and analyze the charge variants present in the tanfanercept-containing ophthalmic composition on the basis of properties thereof, an SP-HP column and a liquid chromatography for separation of protein (FPLC) were used to separate and purify the protein based on a salt concentration. On the basis of charge variant properties, an acidic variant sample A, a main peak sample B and a basic variant sample C were prepared, followed by performing IEF and IEX-HPLC analysis, respectively.

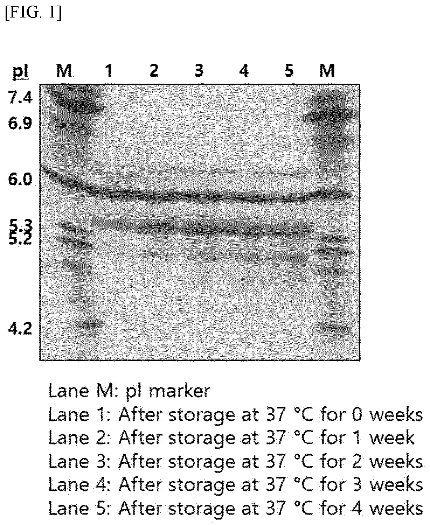

FIG. 1 shows results of isoelectric focusing (IEF) of tanfanercept after storage for 0 to 4 weeks at 37 °C.

From the result of IEF, as shown in FIG. 1A, it was obviously confirmed that, as the storage period is extended, the band at a low pI value becomes darker (or thicker), indicating generation of acidic variants of tanfanercept.

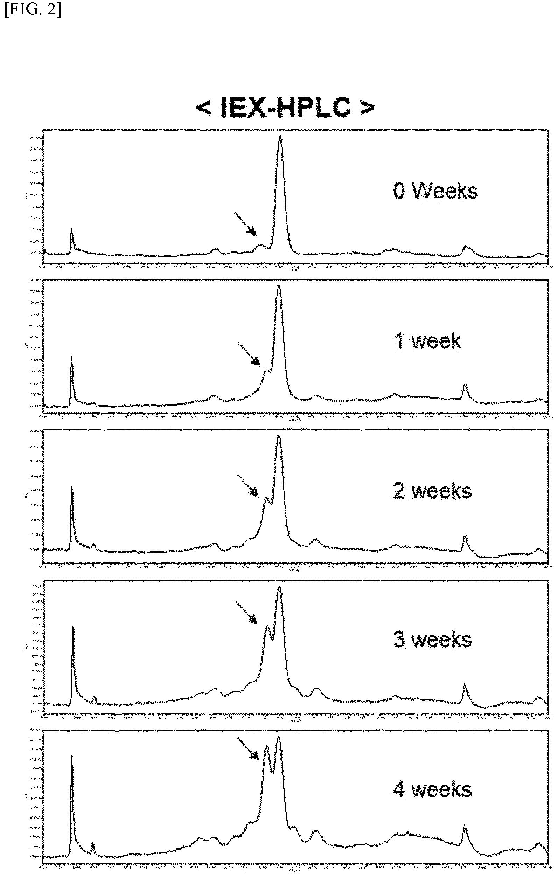

FIG. 2 illustrates results of IEX-HPLC analysis of tanfanercept after storage for 0 to 4 weeks at 37 °C.

Further, from the result of IEX-HPLC, as shown in FIG. 2, it was also confirmed that, as the storage period is extended, an amount of acidic variants is increased.

In order to investigate properties of the charge variant, the acidic variant sample, the main peak sample and the basic variant sample obtained by charge-containing separation using an SP-HP column were subjected to IEF and IEX-HPLC analysis.

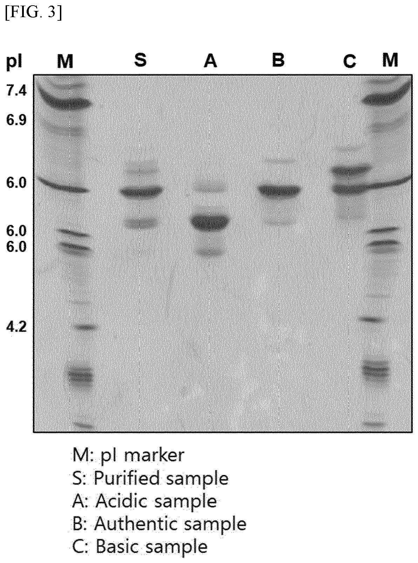

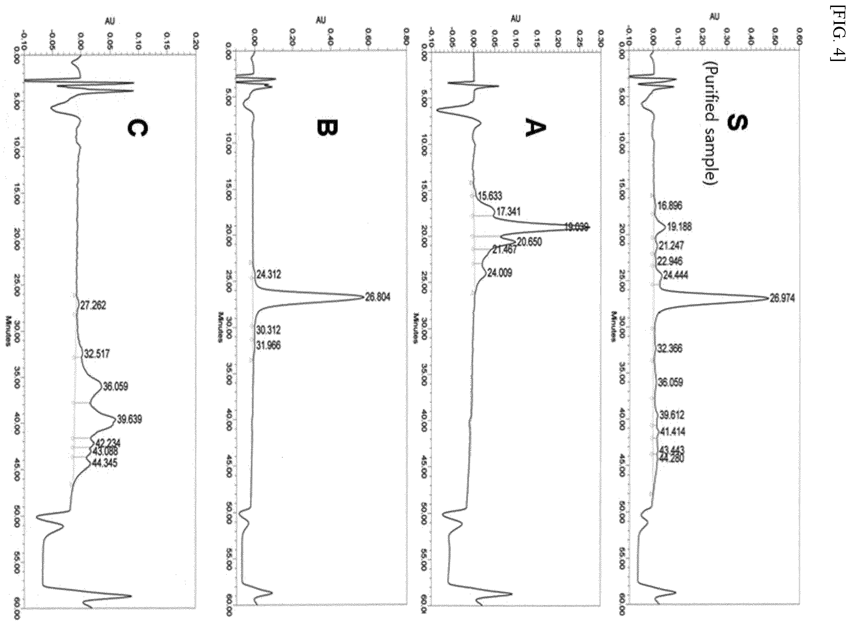

FIG. 3 shows results of IEF analysis of the charge variants. Further, FIG. 4 illustrates results of IEX-HPLC analysis of the charge variants. As shown in FIGS. 3 and 4, the chromatogram result demonstrated that only sample A of the separated acidic variant was eluted before the main peak sample. Also, it could be seen from the IEF result that sample A has a lower pI value than the main peak sample. In addition, the chromatogram result also demonstrated that the basic variant sample, that is, sample B was eluted after the main peak sample. Further, it could be seen from the IEF result that sample B has a slightly higher pI value than the main peak sample.

### Experimental Example 2: Screening of ophthalmic stabilizer

A stabilizer added to a protein composition is generally needed to stably store a formulation until the formulation is administered to a patient. In fact, impurities such as aggregates or charge variants possibly generated during storage can be considerably reduced using the stabilizer, so as to maintain a stable formulation during storage. Therefore, it is absolutely important to select a desirable stabilizer that can stabilize major components of a protein composition, thereby preparing a stable composition. In order to perform stress experiments of stabilizers (40 °C, stored for 4 weeks) and select desired types of the stabilizers that can stabilize tanfanercept as the major component in the present invention, the present inventors first conducted the following experiments.

### 1) Preparation of tanfanercept solution sample

10 mg/mL of tanfanercept solution in 20 mM sodium citrate buffer containing 125 mM sodium chloride was prepared.

### 2) Preparation of citrate phosphate buffer pH 7.0

0.37 g of citric anhydride and 2.58 g of disodium hydrophosphate were added to 900 mL of ultrapure water, followed by thoroughly mixing the same. After titration at pH 7.0 using 37% hydrochloric acid or 40% sodium hydroxide, ultrapure water was added to prepare 1 L of final product.

### 3) Preparation of stabilizer screening solution

Four (4) types of stabilizers (0.149 g of methionine, 0.751 g of glycine, 1.55 g of histidine hydrochloride and 6.84 g of sucrose), respectively, were added to 100 mL of the buffer prepared in item 2), thereby preparing four (4) types of stabilizer-screening compositions at pH 7.0.

**[TABLE 1]**

| | | | | | |
|---|---|---|---|---|---|
| pH 7.0, citrate phosphate buffer | Control | Methionine | Glycine | Histidine hydrochloride | Sucrose |
| | - | - | - | - | - |
| | - | 100 mM | - | - | - |
| | - | - | 100 mM | - | - |
| | - | - | - | 100 mM | - |
| | - | - | - | - | 200 mM |

### 4) Preparation and assessment of sample

After adding 10 mL of the solution for screening ophthalmic stabilizer, which was prepared in item 3), along with tanfanercept to 3.5 kDa centrifugation filter, the sample was centrifuged at 4 °C and 4000 rpm so as to substitute the stabilizer-screening buffer in item 3) for the tanfanercept buffer in item 1). The above procedure was repeated to produce a stabilizer-screening solution containing 1 mg/mL of tanfanercept (sample). The resultant sample was stored at 40°C for 4 weeks, followed by analyzing the sample through IEX-HPLC at 0 weeks and 4 weeks, respectively, so as to analyze physicochemical impurities identified in each sample.

**[TABLE 2]**

| Stabilizer | IEX-HPLC | | | | | | | | |
|---|---|---|---|---|---|---|---|---|---|
| | Start point | | | 4 weeks | | | Change (% peak area) | | |
| | Acidic variant | Main peak | Basic variant | Acidic variant | Main peak | Basic variant | Acidic variant | Main peak | Basic variant |
| Without stabilizer | 0.7 | 97.7 | 1.6 | 34.7 | 50.8 | 14.6 | 33.9 | -46.9 | 13.0 |
| Methioni ne | 0.7 | 98.3 | 1.0 | 35.4 | 50.0 | 14.7 | 34.7 | -48.3 | 13.7 |
| Glycine | 1.0 | 97.1 | 2.0 | 35.6 | 50.2 | 14.2 | 34.7 | -46.9 | 12.2 |
| Sucrose | 0.8 | 97.9 | 1.3 | 32.3 | 54.9 | 12.9 | 31.5 | -43.0 | 11.6 |
| Histidine hydrochl oride | 0.9 | 97.6 | 1.5 | 24.2 | 62.4 | 13.4 | 23.3 | -35.2 | 11.9 |

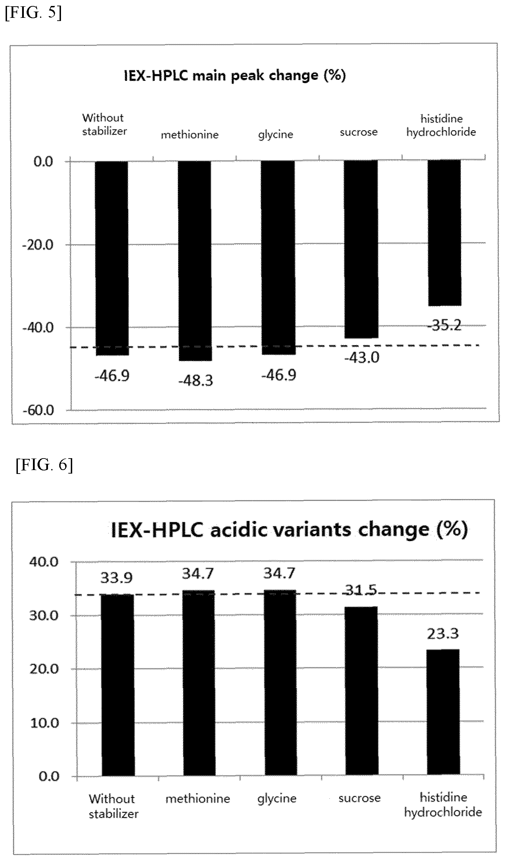

Table 2 and FIGS. 5 to 7 showed IEX-HPLC analysis results of the control group without any stabilizer and the stabilizer screening groups which include methionine, glycine, histidine hydrochloride and sucrose, respectively, as a stabilizer.

Therefore, as shown in Table 2 and FIGS. 5 to 7, it was confirmed that inclusion of sucrose and histidine hydrochloride shows a tendency of generation of basic variants and acidic variants to be reduced. Specifically, in the case of histidine hydrochloride, it could be seen that generation of the acidic variants is significantly reduced as compared to the control group or other stabilizers.

### Experimental Example 3: Preparation of ophthalmic composition at different pH and assessment of stability thereof

Since tears have pH 7.0 to 7.5, it is most preferable to set similar pH conditions for an ophthalmic composition. However, pH may also greatly influence protein stability. Therefore, it was intended to prepare ophthalmic compositions with different pH values and assess stability thereof.

### (1) Preparation of ophthalmic composition

### 1) Preparation of 20 mM citrate phosphate buffer at pH 5.0 to pH 7.0

Citric anhydride and disodium hydrophosphate were added to 400 mL of ultrapure water to correspond to desired pH (in case of pH 5.0/5.5 buffer: 0.62g of citric anhydride and 0.97g of disodium hydrophosphate; in case of pH 6.0/6.5 buffer: 0.43g of citric anhydride and 1.11g of disodium hydrophosphate; in case of pH 7.0 buffer: 0.19g of citric anhydride and 1.29g of disodium hydrophosphate). Further, after titration to pH 5.0 to pH 7.0 using 37% hydrochloric acid or 40% sodium hydroxide in order to correspond to different conditions, ultrapure water was added to prepare 500 mL of final product.

### 2) Preparation of stabilizer-added ophthalmic composition (pH 5.0 to pH 7.0)

To 100 mL of each of five (5) buffers at pH 5.0 to pH 7.0 prepared in item 1), 6.85g of sucrose and 1.55g of histidine were added, thereby preparing five (5) types of 20 mM citrate phosphate buffers at pH 5.0 to pH 7.0, each of which contains 200 mM sucrose, as well as five (5) types of 20 mM citrate phosphate buffers at pH 5.0 to pH 7.0, each of which contains 100 mM histidine. As stabilizer-free experimental groups, the five (5) 20 mM citrate phosphate buffers at pH 5.0 to pH 7.0 prepared in item 1) as described above were directly used as stabilizer-free experimental groups. Therefore, a total of 15 types of buffers at pH 5.0 to pH 7.0 with/without stabilizers was prepared.

### 3) Preparation and assessment of sample

After adding 4 mL of each of the buffers prepared in item 2) as well as tanfanercept, the sample was subjected to centrifugation using a 3.5 kDa centrifugal filter, followed by replacing the existing tanfanercept buffer with the buffer containing a stabilizer at pH 5.0 to pH 7.0. The above procedures were repeated to obtain 15 types of samples containing different stabilizers and having different pH values. The prepared samples were stored at 40 °C for 4 weeks and then analyzed at 0 weeks and 4 weeks, so as to analyze physicochemical impurities identified in each sample.

**[TABLE 3] Preparation of stabilizer-added ophthalmic composition (pH 5.0 to pH 7.0)**

| | Without stabilizer | Histidine | Sucrose |
|---|---|---|---|
| pH 5.0 | 0 mM | 100 mM | 200 mM |
| pH 5.5 | 0 mM | 100 mM | 200 mM |
| pH 6.0 | 0 mM | 100 mM | 200 mM |
| pH 6.5 | 0 mM | 100 mM | 200 mM |
| pH 7.0 | 0 mM | 100 mM | 200 mM |

### (2) Assessment of stability of tanfanercept to pH and stabilizer

### 1) Reverse phase chromatography analysis

RP-HPLC (reverse phase chromatography) is a method for evaluating purity based on polarity of proteins. The present experimental method was conducted using a reverse phase chromatography column, a temperature controller (set to 60 °C), an automatic sampler (set to 4 °C), a UV detector driven at 214 nm and a high performance liquid chromatography (HPLC) system that may maintain a flow rate of 1.0 mL/min.

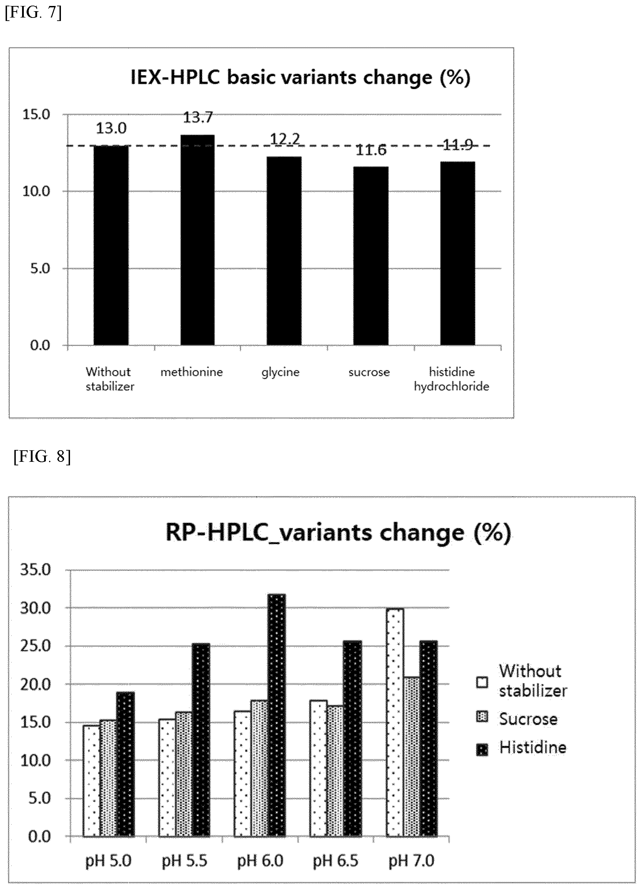

The prepared 15 types of samples were stored under stress conditions (stored at 40 °C) for 4 weeks and then analyzed at 0 weeks and 4 weeks, so as to analyze a change in generation of variants identified in each of the samples. Therefore, as shown in Table 4 and FIG. 8, it was confirmed from the result that the experimental groups at pH 7.0 including sucrose or histidine as a stabilizer exhibit lower rate of generation of variants than the stabilizer-free experimental groups. However, in the case of the experimental groups including histidine as the stabilizer, higher rate of generation of variants at pH 5.0 to pH 6.5 than the control group was demonstrated. Further, in the experimental groups including sucrose as the stabilizer, higher rate of generation of variants at pH 5.0 to pH 6.0 than the control group was demonstrated.

**[TABLE 4]**

| Reverse phase chromatography | RP-HPLC_Change of variant (% peak area) | | | | |
|---|---|---|---|---|---|
| | pH 5.0 | pH 5.5 | pH 6.0 | pH 6.5 | pH 7.0 |
| Without stabilizer | 14.55 | 15.32 | 16.32 | 17.8 | 29.86 |
| Sucrose | 15.28 | 16.33 | 17.86 | 17.18 | 20.93 |
| Histidine | 18.89 | 25.26 | 31.69 | 25.54 | 25.65 |

### 2) Ion exchange high performance liquid chromatography (IEX-HPLC) analysis

IEX-HPLC is used to separate protein using an ion exchanger on the basis of affinity to a fixed phase of a column due to net charge of the protein. The present experimental method was conducted using a cation exchange column, a temperature controller (set to 25 °C), an automatic sampler (set to 4 °C), a UV detector driven at 280 nm and a high performance liquid chromatography (HPLC) system that may maintain a flow rate of 0.7 mL/min.

The prepared 15 types of samples were stored under stress conditions (stored at 40 °C) for 4 weeks and then analyzed at 0 weeks and 4 weeks, followed by comparing changes in generation of acidic/basic variants identified in each of the samples.

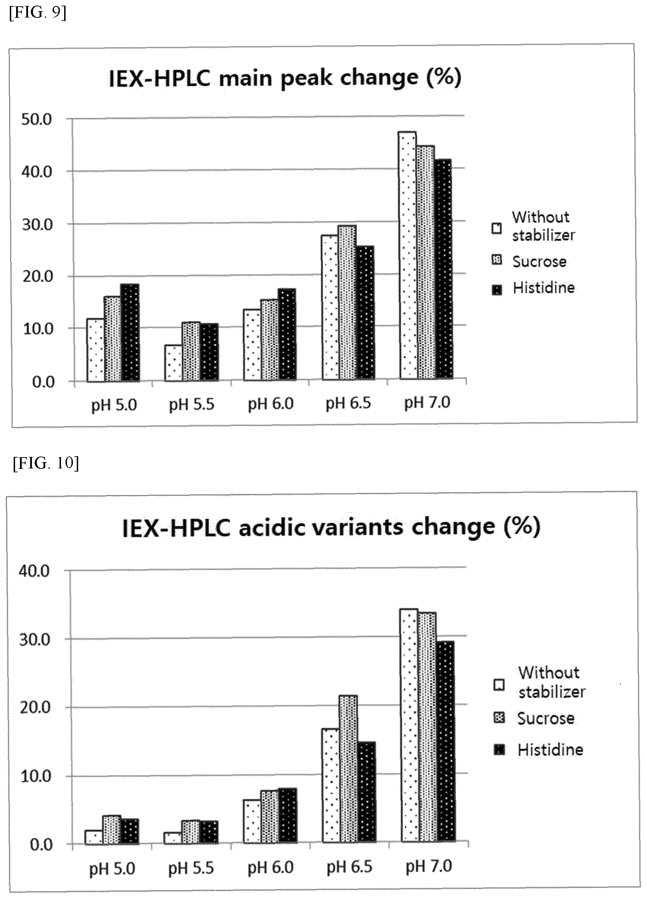

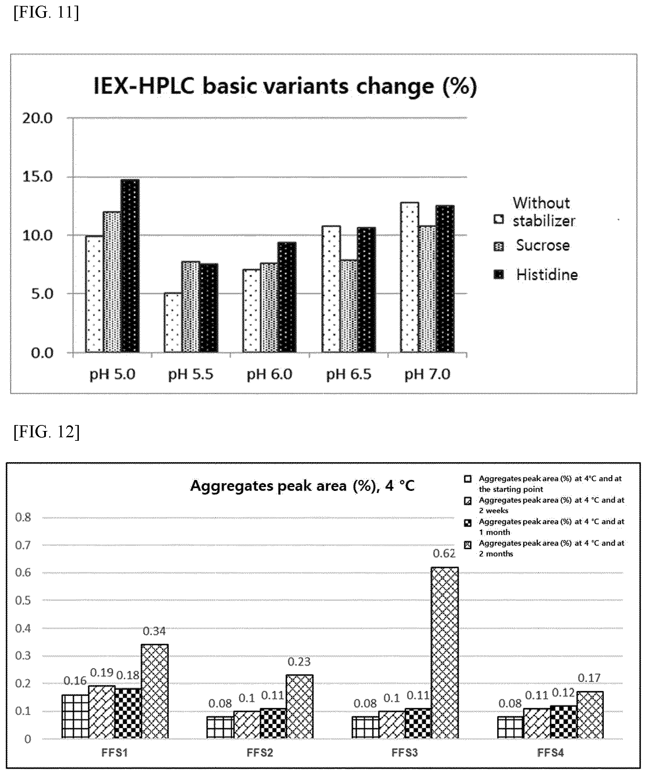

Therefore, as shown in Table 5 and FIGS. 9 to 11, it could be seen that the stabilizer-free group at pH 5.0 to pH 6.5 exhibits better results than the experimental groups containing sucrose or histidine in terms of generation of acidic variants. Specifically, the stabilizer-free group at pH 5.0 to pH 6.0 showed less than 10% of change in generation of acidic variants. For basic variants, the stabilizer-free group at pH 5.0 to pH 6.0 showed better results than the experimental groups containing sucrose or histidine. At pH 6.5 to pH 7.0, the experimental group containing sucrose showed the lowest change in generation of basic variants while the experimental group containing histidine did not have a significant difference from the stabilizer-free group.

**[TABLE 5]**

| IEX-HPLC | Change of acidic variant (% peak area) | | | | | Change of basic variant (% peak area) | | | | | Change of main peak (% peak area) | | | | |
|---|---|---|---|---|---|---|---|---|---|---|---|---|---|---|---|
| | pH 5.0 | pH 5.5 | pH 6.0 | pH 6.5 | pH 7.0 | pH 5.0 | pH 5.5 | pH 6.0 | pH 6.5 | pH 7.0 | pH 5.0 | pH 5.5 | pH 6.0 | pH 6.5 | pH 7.0 |
| Without stabilizer | 2.00 | 1.60 | 630 | 16.60 | 34.00 | 9.90 | 5.10 | 7.10 | 10.80 | 12.80 | 11.90 | 6.70 | 13.40 | 27.40 | 46.90 |
| Sucrose | 4.06 | 328 | 7.60 | 2131 | 33.41 | 12.02 | 7.77 | 7.60 | 7.87 | 10.80 | 16.08 | 11.06 | 1521 | 29.19 | 4421 |
| Histidine | 354 | 3.15 | 7.83 | 1455 | 29.07 | 14.76 | 758 | 9.40 | 10.67 | 1256 | 1830 | 10.73 | 1724 | 2423 | 41.62 |

As a result of analyzing the above measured values, the experimental group containing sucrose or histidine at pH 7.0 showed a tendency of less generation of variants than the stabilizer-free group. On the contrary, when the pH value is decreased to pH 5.5 or pH 6.0, it could be seen that the generation of variants in the experimental group containing sucrose or histidine tends to significantly increase, as compared to the stabilizer-free group.

Combining the experimental results, it could be determined that, contrary to expectations, the experimental group of the tanfanercept-containing ophthalmic composition without any stabilizer at pH 5.5 to pH 6.0, that is, the stabilizer-free group exhibits lowest generation of variants.

In general, the ophthalmic composition may be set to pH 7.0, which is similar to *in vivo* pH. However, stability of an active ingredient in the ophthalmic composition is an essential point for expressing medical efficacy, thereby coming to the conclusion that the tanfanercept-containing ophthalmic composition of the present invention is preferably set to have pH 5.5 to pH 6.0 while not containing sucrose or histidine.

### Experimental Example 4: Assessment of stability to buffer system

In order to compare differences in constitutional composition of buffers and addition/non-addition of two functional excipients while fixing a concentration of tanfanercept, pH, a concentration of sodium chloride, osmotic pressure, etc., four (4) experimental groups were prepared and screened at 4 °C, 25 °C and 40 °C, respectively, so as to select a final formulation. pH of the ophthalmic composition was fixed to pH 5.5 in consideration of the experimental results described above. Further, as a normal buffer generally useable in the above range, sodium acetate (20 mM) and sodium citrate (20 mM) were used to assess tanfanercept relative to the buffer system.

As shown in Table 6 below, four (4) types of tanfanercept-containing ophthalmic compositions were prepared and used to conduct an experiment for stability of tanfanercept.

**[TABLE 6]**

| Component | FFS1 | FFS2 | FFS3 | FFS4 |
|---|---|---|---|---|
| Tanfanercept | 6.25 mg/mL | | | |
| pH | 5.5 | | | |
| Sodium acetate anhydride | 1.44 g | - | - | - |
| Acetic acid | 0.145 g | - | - | - |
| Sodium citrate dianhydride | - | 4.75 g | 4.75 g | 4.75 g |
| Citric acid | - | 0.75 g | 0.75 g | 0.75 g |
| Sodium hyaluronate | - | - | 0.2 mL | - |
| Hypromellose | - | - | | 0.8 mL |
| Sodium chloride | 6.428 g | | | |
| Ultrapure water | 1 L | | | |

By means of analysis of aggregates by SEC-HPLC and analysis of charge variants, the tanfanercept-containing ophthalmic composition was subjected to a stability experiment. The stability experiment was conducted at 4 °C, 25 °C and 40 °C for 2 months in total. At the time of 0 weeks, 2 weeks, 1 month and 2 months, aggregate analysis through SEC-HPLC and charge variant analysis through IEX-HPLC were conducted.

### (1) Aggregates analysis by SEC-HPLC

Size-exclusion high performance liquid chromatography (SEC-HPLC) is typically used to separate protein on the basis of size difference by introducing a fixed phase sample into a column filled with a porous gel. The present experimental method was conducted using a size-exclusion column, a temperature controller (set to 25 °C), an automatic sampler (set to 4 °C), a UV detector driven at 214 nm and a high performance liquid chromatography (HPLC) system that may maintain a flow rate of 0.5 mL/min.

Tables 7 to 9, and FISG. 12 to 14 demonstrated results of SEC-HPLC analysis that was performed while storing the above four (4) types of tanfanercept-containing ophthalmic compositions at 4 °C, 25 °C and 40 °C. From SEC-HPLC analysis results, it could be seen that the higher the temperature, the greater the concentration of aggregates. Specifically, the aggregates were rapidly generated in case of the formulation including a buffer. Among three (3) types of formulations prepared with a citrate buffer, FFS3 was measured to have a high content of aggregates after storage at 4 °C for 2 months. Further, FFS2 without any excipient showed the most stable results in all conditions including 25°C, 40°C, 2 weeks, 1 month and 2 months.

**[TABLE 7] SEC aggregates analysis [4 °C]**

| No. | Peak | % peak area of aggregates | | | |
|---|---|---|---|---|---|
| | Type of sample | 0 | 0.5 | 1 | 2 |
| 1 | FFS1 | 0.16 | 0.19 | 0.18 | 0.34 |
| 2 | FFS2 | 0.08 | 0.10 | 0.11 | 0.23 |
| 3 | FFS3 | 0.08 | 0.10 | 0.11 | 0.62 |
| 4 | FFS4 | 0.08 | 0.11 | 0.12 | 0.17 |

**[TABLE 8] SEC aggregates analysis [25 °C]**

| No. | Peak | % peak area of aggregates | | | |
|---|---|---|---|---|---|
| | Type of sample | 0 | 0.5 | 1 | 2 |
| 1 | FFS1 | 0.16 | 0.38 | 0.46 | 0.77 |
| 2 | FFS2 | 0.08 | 0.19 | 0.22 | 0.31 |
| 3 | FFS3 | 0.08 | 0.18 | 0.22 | 0.36 |
| 4 | FFS4 | 0.08 | 0.22 | 0.28 | 0.46 |

**[TABLE 9] SEC aggregates analysis [40 °C]**

| No. | Peak | % peak area of aggregates | | | |
|---|---|---|---|---|---|
| | Type of sample | 0 | 0.5 | 1 | 2 |
| 1 | FFS1 | 0.16 | 0.98 | 1.40 | 2.24 |
| 2 | FFS2 | 0.08 | 0.45 | 0.61 | 1.03 |
| 3 | FFS3 | 0.08 | 0.44 | 0.63 | 1.14 |
| 4 | FFS4 | 0.08 | 0.56 | 0.81 | 1.37 |

### (2) Acidic variants analysis by IEX-HPLC

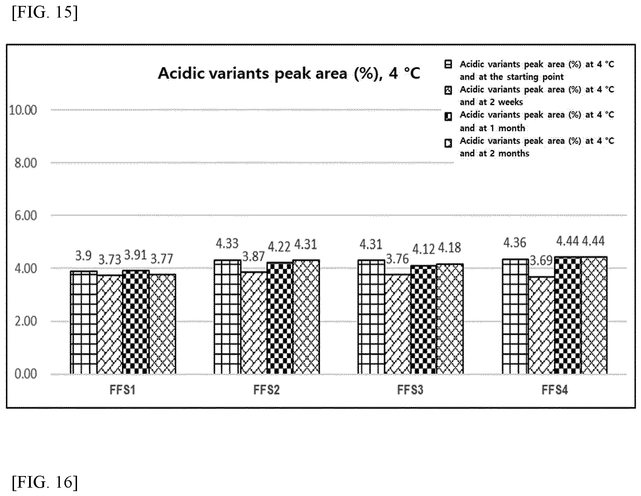

Tables 10 to 12, and FIGS. 15 to 17 demonstrated results of analysis of acidic variants that was performed by IEX-HPLC while storing the above four (4) types of tanfanercept-containing ophthalmic compositions at 4 °C, 25 °C and 40 °C.

**[TABLE 10] IEX-acidic variants [4 °C]**

| No. | Peak | % peak area of acidic variants | | | |
|---|---|---|---|---|---|
| | Type of sample | 0 | 0.5 | 1 | 2 |
| 1 | FFS1 | 3.90 | 3.73 | 3.91 | 3.77 |
| 2 | FFS2 | 4.33 | 3.87 | 4.22 | 4.31 |
| 3 | FFS3 | 4.31 | 3.76 | 4.12 | 4.18 |
| 4 | FFS4 | 4.36 | 3.69 | 4.44 | 4.44 |

**[TABLE 11] IEX-acidic variants [25 °C]**

| No. | Peak | % peak area of acidic variants | | | |
|---|---|---|---|---|---|
| | Type of sample | 0 | 0.5 | 1 | 2 |
| 1 | FFS1 | 3.90 | 3.85 | 4.01 | 4.13 |
| 2 | FFS2 | 4.33 | 4.06 | 4.57 | 4.80 |
| 3 | FFS3 | 4.31 | 3.64 | 4.55 | 4.74 |
| 4 | FFS4 | 4.36 | 4.09 | 4.94 | 4.83 |

**[TABLE 12] IEX-acidic variants [40 °C]**

| No. | Peak | % peak area of acidic variants | | | |
|---|---|---|---|---|---|
| | Type of sample | 0 | 0.5 | 1 | 2 |
| 1 | FFS1 | 3.90 | 6.01 | 4.64 | 9.09 |
| 2 | FFS2 | 4.33 | 6.51 | 6.51 | 12.15 |
| 3 | FFS3 | 4.31 | 6.35 | 7.19 | 13.71 |
| 4 | FFS4 | 4.36 | 6.53 | 6.53 | 11.81 |

### (3) Basic variants analysis by IEX-HPLC

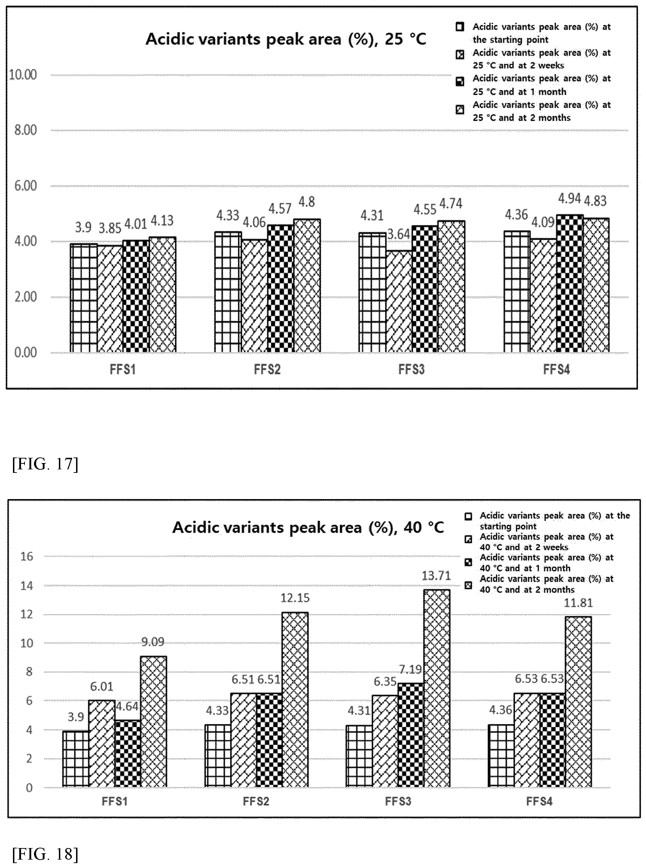

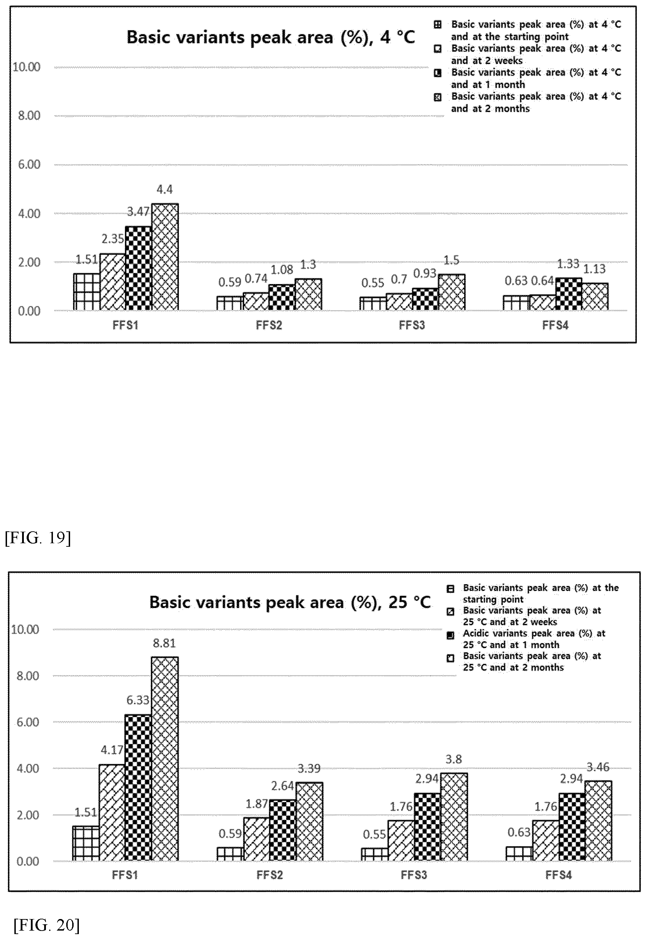

Tables 13 to 15, and FIGS. 18 to 20 demonstrated results of analysis of basic variants that was performed by IEX-HPLC while storing the above four (4) types of tanfanercept-containing ophthalmic compositions at 4 °C, 25 °C and 40 °C.

**[TABLE 13] IEX-basic variants [4 °C]**

| No. | Peak | % peak area of basic variants | | | |
|---|---|---|---|---|---|
| | Type of sample | 0 | 0.5 | 1 | 2 |
| 1 | FFS1 | 1.51 | 2.35 | 3.47 | 4.40 |
| 2 | FFS2 | 0.59 | 0.74 | 1.08 | 1.30 |
| 3 | FFS3 | 0.55 | 0.70 | 0.93 | 1.50 |
| 4 | FFS4 | 0.63 | 0.64 | 1.33 | 1.13 |

**[TABLE 14] IEX-basic variants [25°C]**

| No. | Peak | % peak area of basic variants | | | |
|---|---|---|---|---|---|
| | Type of sample | 0 | 0.5 | 1 | 2 |
| 1 | FFS1 | 1.51 | 4.17 | 6.33 | 8.81 |
| 2 | FFS2 | 0.59 | 1.87 | 2.64 | 3.39 |
| 3 | FFS3 | 0.55 | 1.76 | 2.94 | 3.80 |
| 4 | FFS4 | 0.63 | 1.76 | 2.94 | 3.46 |

**[TABLE 15] IEX-basic variants [40°C]**

| No. | Peak | % peak area of acidic variants | | | |
|---|---|---|---|---|---|
| | Type of sample | 0 | 0.5 | 1 | 2 |
| 1 | FFS1 | 1.51 | 8.79 | 13.29 | 20.66 |
| 2 | FFS2 | 0.59 | 4.96 | 4.96 | 11.77 |
| 3 | FFS3 | 0.55 | 4.97 | 7.74 | 10.70 |
| 4 | FFS4 | 0.63 | 5.03 | 5.03 | 15.00 |

From IEX-HPLC analysis, it could be seen that all formulations show a tendency of acidic and basic variants to be increased under storage conditions at a high temperature of 40 °C, specifically, the acetate buffer (FFS1) formulation exhibits a pattern of significantly increasing the basic variant at all temperatures.

Consequently, in an aspect of generation of aggregates and basic variants, it was confirmed that stability of the acetate buffer (FFS1) is more deteriorated than the citrate buffers (FFS2 to 4). Further, on the basis of constitutional composition of the citrate buffer, when comparing functional excipient-added groups with a group without functional excipient, the group without functional excipient was proved to be most stable. Therefore, in overall consideration of simplification of a product manufacturing process, contamination in the manufacturing process and uniformity, a formulation using the citrate buffer (FFS2) was adopted as a final formulation. However, considering that physiological pH of tears is neutral, pH 6.0 at which patient compliance may be a little higher along with an acceptable level of stability was selected as pH of the final product.

### Preparative Example 1: Production of tanfanercept-containing ophthalmic composition

To 900 ml of ultrapure water, 5.35g of trisodium citrate dihydrate, 0.35g of citric acid anhydride and 7.3g of sodium chloride were added and completely dissolved, thereby preparing a buffer. After confirming pH of the buffer as pH 6.0 ± 0.1, an amount of the buffer was adjusted to reach a final solution volume of 1 L using a graduated cylinder and then filtered through a 0.22 µm bottle top filter system.

Then, tanfanercept was added to the prepared buffer to produce 0.25% tanfanercept-containing eye drop composition.

**[TABLE 16]**

| Component | Content (mg/ml) |
|---|---|
| Tanfanercept | 2.5 |
| Trisodium citrate, dihydrate | 5.35 |
| Citric acid anhydride | 0.35 |
| Sodium chloride | 7.31 |
| Sodium hydroxide | As required |
| Hydrochloric acid | As required |
| Ultrapure water | Q.S. |

### Experimental Example 5: Assessment of stability of tanfanercept-containing ophthalmic composition

The tanfanercept-containing ophthalmic composition according to Preparative Example 1 was subjected to assessment of stability.

A long term storage condition was set to 5 °C (humidity not adjusted), and an accelerated condition was set to 25 °C/60% RH. The composition in Preparative Example 1 was stored under these conditions, and then, was subjected to aggregate analysis through SEC-HPLC and charge variant analysis through IEX-HPLC.

**[TABLE 17]**

| Analysis method | Storage condition | Start | 3 months | 6 months | 9 months | 12 months | 18 months | 24 months | 36 months |
|---|---|---|---|---|---|---|---|---|---|
| SEC-HPLC (aggregate , %) | Long term storage | 0.1 | 0.2 | 0.1 | 0.2 | 0.2 | 0.2 | 0.2 | 0.3 |
| | Accelerated | 0.1 | 0.3 | 0.4 | | | | | |

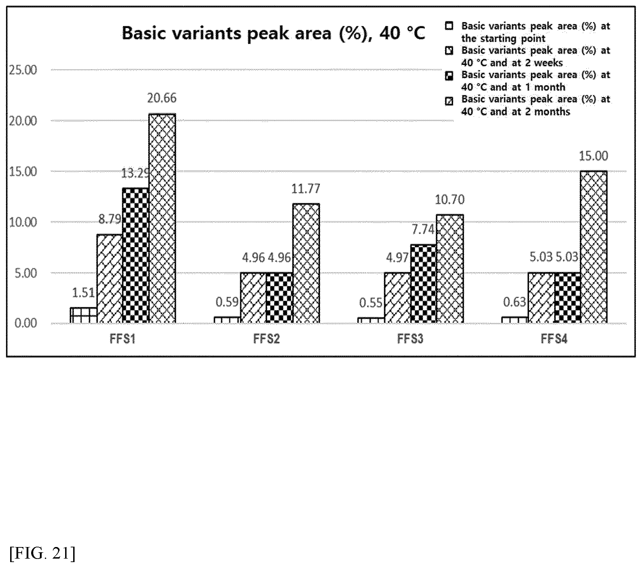

Table 17 and FIG. 21 showed results of the aggregate analysis through SEC-HPLC. As shown in Table 17 and FIG. 21, the composition in Preparative Example 1 was confirmed to be stable since aggregates were formed within 5% when the composition was stored for 3 years under a long term storage condition at 5°C. Further, when the composition was stored for 6 months under accelerated conditions at 25°C/60% RH, generation of aggregates within 5% was demonstrated.

**[TABLE 18]**

| Analysis method | Storage condition | Start | 3 months | 6 months | 9 months | 12 months | 18 months | 24 months | 36 months |
|---|---|---|---|---|---|---|---|---|---|
| IEX-HPLC (acidic variant, %) | Long term storage | 3.2 | 3.3 | 3.3 | 2.7 | 3.3 | 2.1 | 2 | 3.6 |
| | Accelerated | 3.2 | 4.1 | 4.1 | | | | | |
| IEX-HPLC (basic variant, %) | Long term storage | 1.1 | 1.5 | 1.4 | 2.6 | 1.9 | 3.2 | 5.1 | 8 |
| | Accelerated | 1.1 | 3.7 | 5.9 | | | | | |

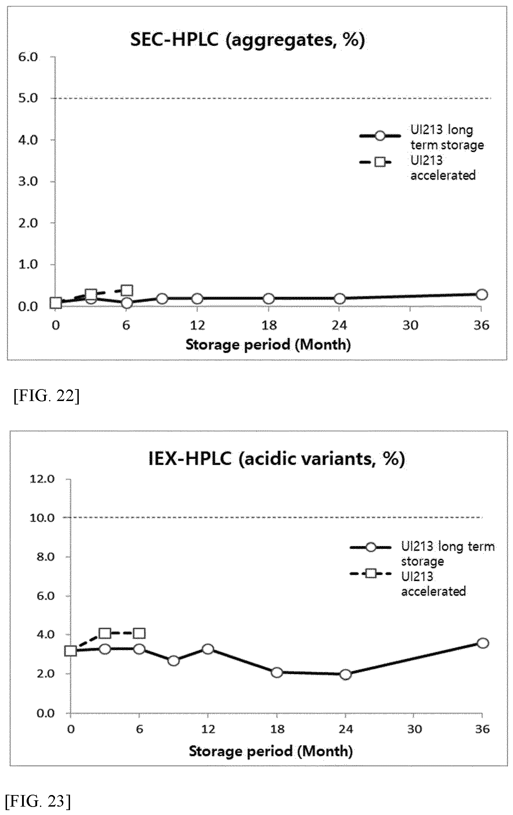

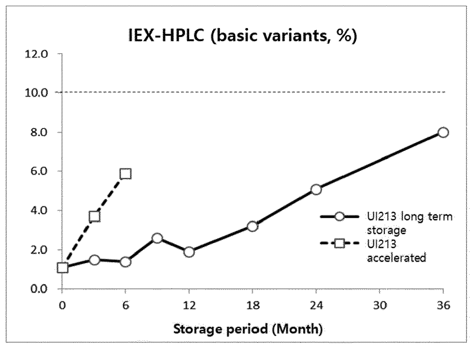

Table 18 and FIGS. 22 to 23 show results of charge variant analysis through IEX-HPLC. As shown in Table 18 and FIGS. 22 to 23, the composition in Preparative Example 1 was confirmed to be stable since charge variants were formed within 5% during storage when the composition was stored for 3 years under a long term storage condition at 5 °C. Further, the basic variants were also formed within 10%, thereby demonstrating stability during storage.

In addition, when the composition was stored for 6 months under accelerated conditions at 25 °C/60% RH, formation of acidic variants within 10% was demonstrated. Further, the basic variants were formed within 10%, thereby demonstrating stability during storage.

## Claims

1. A stable tanfanercept-containing ophthalmic composition, comprising:
tanfanercept and a buffer system of pH 5.0 to pH 6.5,
which does not substantially comprise a stabilizer.

2. The composition of claim 1, wherein the tanfanercept is contained at 0.01 %(w/v) to 10 %(w/v).

3. The composition of claim 1, wherein the pH of the buffer system is pH 5.0 to pH 6.0.

4. The composition of claim 1, wherein the pH of the buffer system is pH 5.5 to pH 6.0.

5. The composition of claim 1, wherein the buffer system comprises one or two or more buffers selected from the group consisting of a phosphate buffer, a histidine buffer, an acetate buffer, a succinate buffer, a citrate buffer, a glutamate buffer and a lactate buffer.

6. The composition of claim 1, wherein the buffer system is a citrate buffer system, a phosphate buffer system or a citrate-phosphate buffer system.

7. The composition of claim 6, wherein the citrate buffer system comprises trisodium citrate and citric acid as buffers.

8. The composition of claim 1, wherein the buffer system comprises a 5 to 50 mM buffer.

9. The composition of claim 1, further comprising
an isotonic agent.

10. The composition of claim 9, wherein the isotonic agent is sodium chloride.

11. The composition of claim 1, wherein the ophthalmic composition has a tanfanercept charge variant in an amount of 20% or less after 6-month storage under accelerated conditions.

12. The composition of claim 1, wherein the ophthalmic composition has a tanfanercept basic variant in an amount of 10% or less after 6-month storage under accelerated conditions.

13. The composition of claim 1, wherein the ophthalmic composition has a tanfanercept acidic variant in an amount of 10% or less after 6-month storage under accelerated conditions.

14. The composition of claim 1, wherein the ophthalmic composition has a tanfanercept charge variant in an amount of 20% or less after 36-month storage under long-term storage conditions.

15. The composition of claim 1, wherein the ophthalmic composition has a tanfanercept basic variant in an amount of 10% or less after 36-month storage under long-term storage conditions.

16. The composition of claim 1, wherein the ophthalmic composition has a tanfanercept acidic variant in an amount of 10% or less after 36-month storage under long-term storage conditions.

17. The composition of claim 1, wherein the osmolality of the ophthalmic composition is 260 to 320 mOsm/kg.
